Europäisches Patentamt

European Patent Office

Office européen des brevets

(19)

(11) EP 0 850 663 B1

(12) FASCICULE DE BREVET EUROPEEN

(45) Date de publication et mention
de la délivrance du brevet:
**02.03.2005 Bulletin 2005/09**

(51) Int Cl.⁷: **A61N 1/372**

(21) Numéro de dépôt: **97403147.8**

(22) Date de dépôt: **23.12.1997**

(54) **Dispositif médical implantable actif, et son programmateur externe, à ajustement dynamique de la puissance d'émission**

Aktive implantierbare medizinische Vorrichtung und ihr externes Programmiergerät, mit dynamischer Einstellung der Sendeleistung

Active implantable medical device and its external programmer, with dynamic adjustemnt of the transmitting power

(84) Etats contractants désignés:
**BE CH DE DK ES FR GB IE IT LI LU NL PT SE**

(30) Priorité: **24.12.1996 FR 9615909**

(43) Date de publication de la demande:
**01.07.1998 Bulletin 1998/27**

(73) Titulaire: **ELA MEDICAL**
**F-92541 Montrouge (FR)**

(72) Inventeurs:
• **Deschamp, Hervé**
**92150 Suresnes (FR)**
• **Garcia, Luc**
**38330 Saint Ismier (FR)**
• **Basile, François**
**92160 Antony (FR)**
• **Lee, Chik Yam**
**94110 Arcueil (FR)**

(74) Mandataire: **Dupuis-Latour, Dominique et al**
**SEP Pagenberg & Associés**
**14 boulevard Malesherbes**
**75008 Paris (FR)**

(56) Documents cités:
**WO-A-92/07620          WO-A-95/16393**
**WO-A-96/22125          US-A- 4 531 523**
**US-A- 4 868 795          US-A- 5 168 871**
**US-A- 5 522 865          US-A- 5 562 713**

## Description

La présente invention concerne le domaine des dispositifs médicaux implantés actifs.

Les dispositifs médicaux implantés actifs comprennent notamment les stimulateurs cardiaques, les défibrillateurs, les appareils neurologiques, les pompes de diffusion de substances médicales et les implants cochléaires.

Ces appareils, une fois mis en place, sont programmés de l'extérieur au moyen d'une console distante appelée "programmateur". La vérification des paramètres de l'implant ou la transmission d'informations enregistrées par celui-ci s'effectue par voie électromagnétique, technique appelée "télémétrie" dans le domaine en question. Cette console est munie d'un organe récepteur qui est placé en regard du site de l'implant et qui comprend une bobine qui capte le champ magnétique en provenance de l'appareil implanté.

Inversement, le programmateur est susceptible d'envoyer des informations vers l'implant par voie électromagnétique, en faisant osciller des courants dans une bobine du programmateur, ce qui va provoquer une apparition de tensions aux bornes de la bobine de réception de l'implant ; ces tensions sont captées et décodées par l'implant. Inversement, les signaux émis par l'implant sont captés par la bobine, amplifiés, filtrés, numérisés et décodés, ce qui permet de réaliser ainsi une transmission dans les deux sens entre implant et programmateur.

Les champs électromagnétiques émis aussi bien par le programmateur que par l'implant présentent une amplitude qui varie fortement avec la distance par rapport à l'appareil émetteur. Ainsi, par exemple, l'amplitude du champ magnétique oscillant émis par certains implants peut varier d'un facteur dont l'ordre de grandeur est proche de vingt, selon que le champ est recueilli à un centimètre ou à sept centimètres de l'implant, distances qui sont des valeurs typiques couramment rencontrées dans la pratique.

Or, chaque fois qu'un appareil (implant ou programmateur) émet des signaux en faisant circuler des courants dans sa bobine d'émission, il consomme une certaine énergie. En particulier, il est connu que pour un circuit oscillant donné l'énergie consommée pour entretenir un nombre d'oscillations donné varie proportionnellement au carré de l'amplitude du courant (ou de la tension) caractéristique de ces oscillations.

Jusqu'à présent, pour garantir une émission correcte, on faisait fonctionner le programmateur à pleine puissance, en prévoyant dans l'implant des circuits suppresseurs (*clamping*), mis en oeuvre si la puissance reçue est trop forte. Ces circuits suppresseurs n'ont cependant qu'une fonction de sécurité contre les surtensions, et il n'est généralement pas souhaitable de les mettre en oeuvre car ils conduisent à une dégradation de la transmission des signaux et du fonctionnement de l'implant.

L'un des buts de l'invention est de proposer un moyen pour faire varier l'énergie d'émission en l'adaptant aux possibilités de réception de l'autre élément, en particulier en l'adaptant à la distance séparant émetteur et récepteur. En adaptant ainsi la puissance d'émission, on optimise corrélativement l'énergie consommée, et on limite donc celle-ci au strict minimum requis pour permettre une transmission de qualité, c'est-à-dire avec un rapport signal/bruit satisfaisant.

On verra également que l'invention permet de tenir compte des variations dynamiques de la distance entre émetteur et récepteur, par exemple les variations dues à la respiration du patient ou au léger déplacement de la tête de programmation tenue par le praticien. L'invention, comme on le verra, permet de suivre ces variations de distance en modifiant de façon continue la puissance d'émission de manière à maintenir celle-ci à une valeur optimale en dépit des variations de niveau requises.

La conséquence première de l'adaptation du niveau de puissance est une réduction significative de la consommation des circuits d'émission. Ainsi, si un implant est par exemple à même de détecter un programmateur émettant vers lui à une distance de sept centimètres, alors le programmateur pourra être détecté à un centimètre en envoyant vers l'implant des signaux d'amplitudes vingt fois plus faibles que lorsque la distance est de sept centimètres. L'énergie dépensée dans le programmateur pourra être réduite ainsi d'un facteur quatre cents. Cette réduction de consommation est particulièrement intéressante dans le cas des programmateurs portatifs autonomes fonctionnant sur batterie, où il est nécessaire de gérer au mieux la consommation de l'ensemble afin de procurer une autonomie suffisante.

Les mêmes considérations s'appliquent, *mutatis mutandis,* au cas de l'émission depuis l'implant vers le programmateur. Dans ce cas, la consommation d'énergie plus faible se traduit par un allongement de la durée de vie de l'implant, ce qui est, on le comprendra, particulièrement important. Egalement, en fin de vie de la pile de l'implant, l'appel de courant important d'une émission à pleine puissance peut provoquer une chute de tension notable (typiquement 100 mV pour un appel de courant de 10 µA sur une charge de 10 kΩ), ce qui est particulièrement critique si l'on se trouve aux limites inférieures des possibilités de l'implant.

Le document US-A-5 522 865 décrit un appareil selon les préambules des revendications 1 et 2.

L'invention est définie dans les revendications 1 et 2.

On va maintenant décrire une mise en oeuvre de l'ensemble de l'invention.

La figure 1 illustre, schématiquement, l'ensemble implant et programmateur selon l'invention.
La figure 2 est un schéma par blocs des différents organes d'émission, de détection et de contrôle de l'implant et du programmateur selon l'invention, montrant également les divers signaux échangés pour permettre l'adaptation des niveaux d'émission.
La figure 3 est un détail du bloc d'émission/détection de l'implant.
La figure 4 est un détail du bloc de détection à sensibilité variable de l'implant.

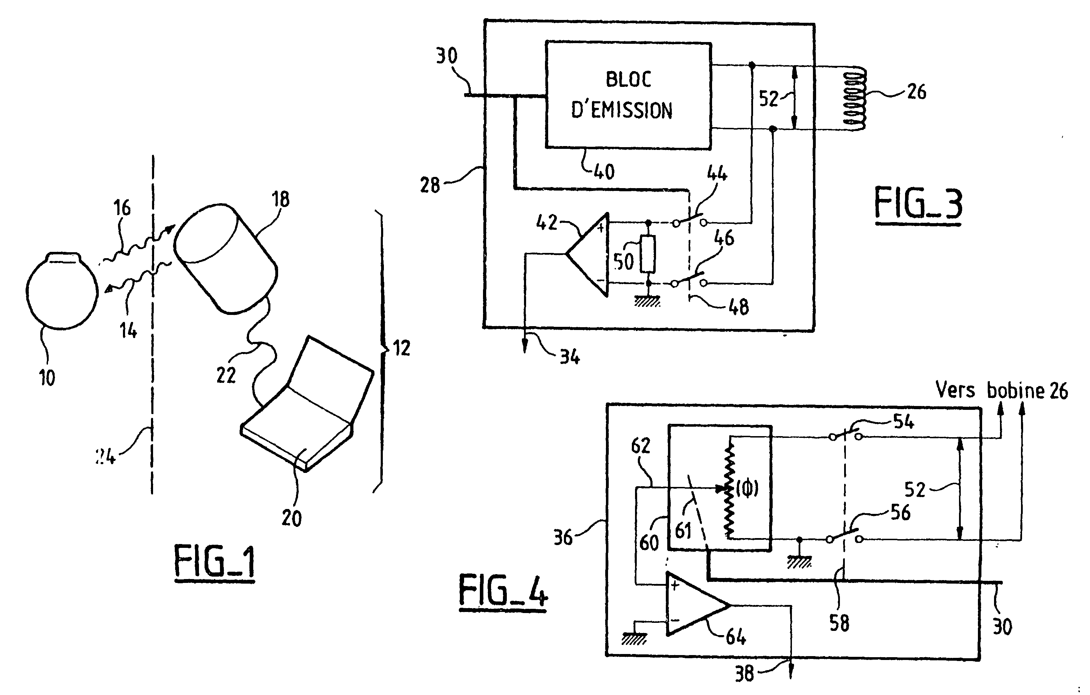

Sur la figure 1, la référence 10 désigne un "dispositif autonome" au sens large défini plus haut, dans cet exemple un dispositif médical implanté chez un patient. Cet implant 10 peut entrer en communication bidirectionnelle avec un programmateur 12 duquel il reçoit des messages 14, par exemple des requêtes d'interrogation ou des ordres de réglage de certains paramètres de fonctionnement de l'implant, et vers lequel il émet des signaux 16, par exemple des informations conservées dans une mémoire interne de l'implant.

Le programmateur 12 comporte, essentiellement, une unité d'émission et de réception 18 ou "tête de programmation" (par exemple un dispositif du type comparable à celui décrit dans le EP-A-0 661 077 auquel on pourra se reporter pour de plus amples détails sur la manière dont les signaux sont recueillis ou traités), reliée à un ordinateur portable 20 via un câble de liaison 22.

L'implant 10 et le programmateur 12 sont séparés par un entrefer incluant de l'air et des tissus humains (la référence 24 symbolise la barrière cutanée), cette distance pouvant varier typiquement, comme indiqué plus haut, de 1 à 7 centimètres.

*Structure générale de l'implant*

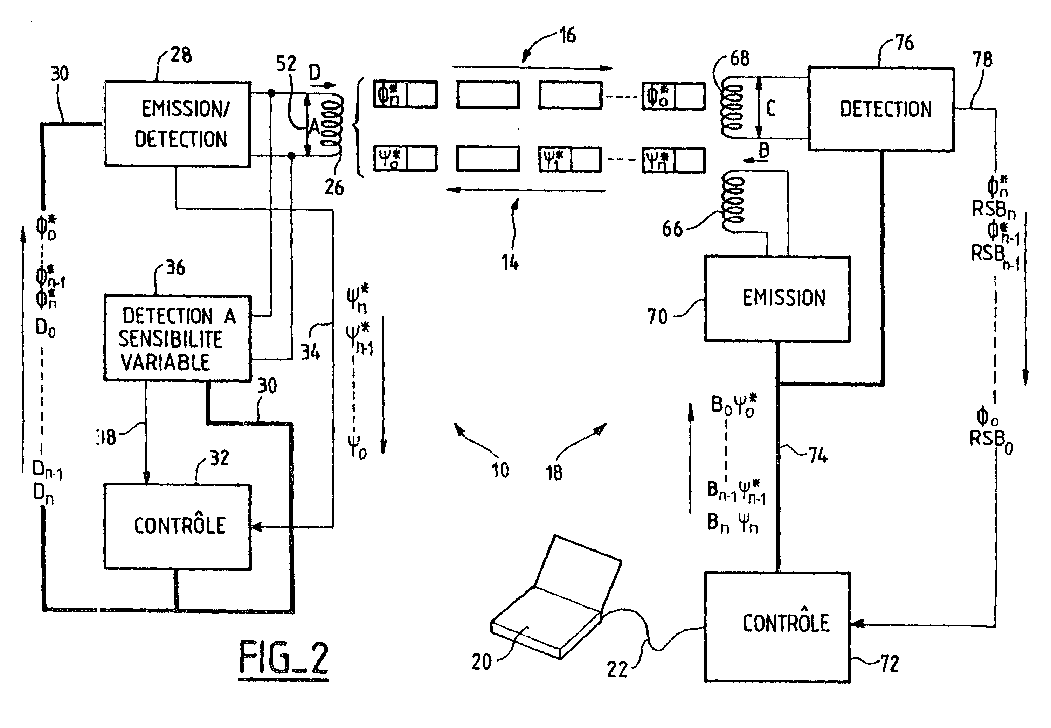

Comme cela a été illustré sur la partie gauche de la figure 2, l'implant comporte une bobine, qui est généralement une bobine unique 26 servant à la fois à la réception des messages 14 et à l'émission des messages 16, ces messages n'existant pas simultanément (communication en alternat).

L'implant 10 comporte un bloc 28 d'émission/détection comprenant à la fois des moyens pour générer dans la bobine 26 les courants pour l'émission des messages 16, et des moyens pour détecter les messages 14. Le fonctionnement de ce bloc 28, en particulier le basculement du mode d'émission au mode de détection, est contrôlé par les signaux d'un bus 30, signaux qui sont générés par un bloc logique de contrôle 32. Le bloc d'émission/détection 28 délivre par 34 au bloc de contrôle 32 le résultat de la détection qu'il a opéré.

L'implant 10 comporte également, de manière caractéristique de l'invention, un bloc 36, également dévolu à la détection, qui transmet par 38 le résultat de sa détection au bloc de contrôle 32.

Le bloc d'émission/détection 28 est représenté plus en détail sur la figure 3. Il comporte un bloc d'émission 40, contrôlé par le bus 30, qui permet de générer dans la bobine 26 les courants nécessaires à l'émission des messages en direction du programmateur ; l'une des fonctions des signaux du bus 30 est de contrôler l'amplitude des courants ainsi générés.

Lorsque le bloc 28 est en mode de réception, le bloc d'émission 40 est rendu inopérant par le bus 30, et les bornes de la bobine 26 sont connectées aux deux entrées d'un comparateur 42. Cette connexion est réalisée sur ordre du bloc de contrôle 32 via le bus 30 et deux interrupteurs 44, 46, par exemple des interrupteurs contrôlés par un signal logique 48 issu du bus de contrôle 30. Une résistance 50, montée entre les deux entrées du comparateur 42, permet d'éviter des oscillations intempestives du système.

La sortie du comparateur, qui correspond à la ligne du signal 34 précitée, est appliquée au bloc de contrôle 32, en lui indiquant la présence éventuelle d'une tension 52 générée aux bornes de la bobine 26 par les champs électromagnétiques porteurs des messages 14.

Si l'on appelle σ le seuil du comparateur 42, la détection des messages incidents peut s'effectuer dès que A > σ, A désignant l'amplitude du signal 52 qui prend naissance aux bornes de la bobine 26.

Le bloc 36 de détection à sensibilité variable est décrit plus en détail sur la figure 4. Ce bloc entre en fonction lors de la fermeture des interrupteurs 54 et 56, commandés par un signal 58 issu du bus 30. Cette opération met en communication la bobine 26 avec un atténuateur programmable 60, que l'on peut fonctionnellement assimiler à un potentiomètre. L'atténuateur programmable 60 multiplie le signal d'entrée 52 par un facteur $\Phi$ ($\Phi < 1$), déterminé par un code numérique 61 transmis du le bloc de contrôle 32 par le bus 30. Le signal divisé 62 est présenté à l'entrée d'un comparateur 64 semblable au comparateur 42 du bloc 28, donc avec le même seuil de déclenchement σ. L'autre entrée du comparateur est reliée à un potentiel fixe, par exemple à la masse et sa sortie constitue le signal 38 précité qui sera analysé par le bloc de contrôle 32.

*Structure générale du programmateur*

Comme cela a été illustré sur la partie droite de la figure 2, le programmateur 18 comporte une bobine d'émission 66 et une bobine de réception 68. Cette configuration, qui est la plus courante, n'est cependant pas limitative et l'on pourrait aussi bien concevoir un programmateur comprenant une seule bobine d'émission/détection, comme dans le cas de l'implant.

Lorsqu'un message 14 est envoyé vers l'implant, un courant oscillant circule dans la bobine d'émission 66. Ce courant est généré par un bloc d'émission 70, lui-même commandé par un bloc de contrôle 72 via un bus 74. Si l'on désigne par B l'amplitude du courant qui circule dans la bobine 66 lors de l'émission d'un message 14, l'amplitude A de la tension induite aux bornes de la bobine de réception 26 de l'implant sera proportionnelle à B, pour des raisons de linéarité bien connues de l'homme de l'art.

Pour la réception des signaux 16 émis par l'implant, le programmateur met en oeuvre un bloc de détection 76 relié à la bobine de réception 68, et envoie en 78 les signaux ainsi détectés au bloc de contrôle 72 qui les traite.

*Contrôle de la puissance émise par le programmateur*

Pour réduire la consommation dans le programmateur, l'invention utilise la transmission bidirectionnelle des données, à savoir la succession de messages 14 et 16 échangés entre implant et programmateur.

On va tout d'abord décrire la manière dont l'invention est mise en oeuvre pour adapter l'énergie émise par le programmateur, on verra ensuite que l'invention peut être mise en oeuvre de façon symétrique, pour la réduction de la puissance d'émission de l'implant.

Supposons tout d'abord que la réception soit effective, c'est-à-dire que l'on ait :

$$A > \sigma.$$

En même temps que les messages 14 sont correctement reçus par le bloc d'émission/détection 28, le bloc de contrôle 32 propose au bloc à sensibilité variable 36 divers facteurs de multiplication $\Phi$, décroissants, ceci jusqu'à l'obtention d'un facteur $\Phi^*$ déclenchant l'arrêt des basculements du comparateur 64.

On a alors :

$$\Phi^*.A = \sigma$$

Cette valeur de $\Phi^*$ est envoyée de l'implant au programmateur par un message 16.

Les messages 16 sont captés par la bobine 68 du programmateur, décodés par le bloc de détection 76. Ce dernier délivre au bloc de contrôle 72, via la ligne 78, d'une part le contenu des messages reçus, avec notamment les valeurs $\Phi^*$, et, d'autre part, la valeur RSB du rapport signal/bruit, valeur qui est obtenue par des mesures successives du bruit, puis du signal, par le bloc de détection 76. Le bloc de contrôle 72 envoie vers l'ordinateur portable 20 les parties des messages 16 qui lui sont destinés (typiquement, les contenus des registres et de la mémoire de l'implant), via le câble 22.

On va maintenant décrire comment le bloc de contrôle 72 commande, via le bus 30, l'amplitude B des courants oscillants émis par le bloc 70.

Pour cela, on recueille dans un message 16 la valeur du facteur $\Phi^*$, afin d'en déduire une valeur B minimale assurant une bonne qualité de transmission. Cette valeur, déduite de $\Phi^*$, assurera la transmission pour un minimum d'énergie consommée.

En effet, si pour une distance de transmission donnée une amplitude B dans la bobine 66 du programmateur induit une tension A aux bornes de la bobine 26 de l'implant, et que l'on a A > $\sigma$, avec $\Phi^*.A = \sigma$ ($\Phi^* < 1$), alors une amplitude $\Phi^*.B$ dans la bobine 66 induira une tension d'amplitude $\Phi^*.A = \sigma$ dans la bobine 26.

Comme cela ressort de la définition de $\Phi^*$, cette amplitude $\Phi^*.A$ est juste suffisante pour faire basculer le comparateur 42. Le bloc de contrôle 72 imposera donc, à la réception d'une valeur $\Phi^*$, une amplitude $\Phi^*.(1+s).B$, où s est un facteur de sécurité positif qui garantit aux bornes du comparateur 42 une amplitude $(1+s).\sigma$ supérieure au minimum requis et évite un basculement inverse du comparateur.

Pour s'ajuster aux variations de distance, notamment dues à la respiration du patient, qui interviennent continûment au cours d'une même session d'interrogation d'un implant, la mesure de $\Phi^*$ et le calcul de B sont effectués périodiquement. L'intervalle de temps entre deux mesures doit être faible par rapport à la vitesse caractéristique du déplacement humain (cette vitesse est, typiquement, d'au plus 1 mm en 0,1 s, soit 1 cm/s), ce qui est possible dans la mesure où la tenue de la tête de programmation face à l'implant s'effectue manuellement.

On nommera $\Phi_0^*$, $\Phi_1^*$ ... $\Phi_n^*$ ... la suite des facteurs $\Phi^*$ mesurés successivement par l'implant, et $B_0$, $B_1$ ... $B_n$ ... la suite des amplitudes des courants émis par la bobine 66 du programmateur.

Au début de la session, on choisit une amplitude $B_o$ la plus élevée disponible dans le programmateur.

Les relations de dépendance entre les facteurs $\Phi_n^*$ s'obtiennent par calculs itératifs. Ainsi, si $B_n$ est l'amplitude au $(1+n)^{ième}$ réglage et $\Phi_n^*$ le $(1+n)^{ième}$ facteur calculé, on a :

$$B_{n+1} = \Phi_n^*.(1+s).B_n.$$

Le système devient stable lorsque :

$$B_{n+1} = B_n,$$

soit :

$$\Phi_n^* = 1/(1+s) \; \forall \; n.$$

Pour une même distance entre la tête de programmation et l'implant, il existe un rapport S(d) ou "facteur de couplage" fixe entre l'amplitude B du courant dans la bobine 66 et l'amplitude A de la tension induite aux bornes de la bobine 26. Si d désigne la distance, on a :

$$A = R(d).B,$$

et l'on peut utiliser cette relation pour calculer la suite des $\Phi_n{}^*$.

Au début de la session, on a :

$$A_0 = R(d).B_0$$

et

$$\Phi_0{}^* = \sigma/A_0.$$

L'amplitude B du courant dans la bobine 66 calculée à la première itération est donnée par :

$$B_1 = \Phi_0{}^*.(1+s).B_0.$$

D'où une nouvelle amplitude $A_1$ de la tension aux bornes de la bobine 26 :

$$A_1 = A_0.(B_1/B_0) = A_0.\Phi_0{}^*.(1+s),$$

ce qui donne le second facteur $\Phi^*$:

$$\Phi_1{}^* = \sigma/A_1 = \Phi_0{}^*.(A_0/A_1) = 1/1+s.$$

Dans cette hypothèse (distance invariante entre implant et programmateur), le système est donc stable dés le première itération.

Dans l'hypothèse où le facteur de couplage R(d) varie, si l'intervalle de temps entre deux mesures est faible par rapport à la vitesse caractéristique du déplacement humain (cf. *supra*), le système se stabilisera, également.

Appelons R(d) le rapport entre $A_n$ et $B_n$ et R(d).(1+f) le rapport entre $A_{n+1}$ et $B_{n+1}$, f désignant la proportion dont a varié le rapport A/B (variation due à un mouvement relatif de la tête par rapport à l'implant).

Pour un système stable, on doit avoir :

$$A_n = \sigma(1+s),$$

donc :

$$B_{n+1} = B_n$$

et

$$A_{n+1} = A_n(1+f).$$

Il vient:

$$\Phi_{n+1}{}^* = \sigma/A_{n+1} = 1/(1+s)(1+f),$$

donc:

$$B_{n+2} = B_{n+1}.\Phi_{n+1}{}^*.(1+s) = B_{n+1}.(1+f),$$

et

$$A_{n+2} = R(d).(1+f)B_{n+2} = R(d).B_{n+1} = R(d).B_n = A_n.$$

L'amplitude se stabilise ainsi en une itération à partir de n+1.

Les expressions ci-dessus permettent de calculer le facteur de sécurité s en fonction de la vitesse relative V de la tête de programmation par rapport à l'implant, dés lors que l'on peut mesurer le profil R(d), ainsi que V.

Le gain d'énergie par rapport au cas où il n'y a pas de régulation est :

$$(B_{n\ stable}/B_o)^2.$$

Comme on l'a démontré plus haut, le système est stable pour $\Phi_1{}^*$, c'est-à-dire pour $B_2$, et l'on a :

$$B_2 = (1+s)^2.\Phi_1{}^*.\Phi_0{}^*.B_0 = [(1+s)^2/(1+s)].[\sigma/A_0].B_0.$$

L'énergie nécessaire est donc réduite d'un facteur :

$$\{A_0 / [\sigma(1+s)]\}^2$$

Typiquement, ce facteur peut être de l'ordre de cent, si la tête de programmation et l'implant sont séparés d'une distance très inférieure à la distance maximale de transmission.

*Contrôle de la puissance émise par l'implant*

Un procédé semblable à celui que l'on vient de décrire pour le programmateur peut être appliqué à l'implant.

Cette fois, c'est le programmateur qui contrôle l'énergie émise par l'implant via des messages 14 émis en direction de ce dernier.

Le bloc 28 d'émission/détection génère dans la bobine 26 de l'implant un courant d'amplitude D lors de l'envoi d'un message 16 vers le programmateur. Ce courant, selon l'invention, est ajusté en fonction d'une consigne fournie par le bloc de contrôle 32 via le bus 30.

Lorsque les messages 16 sont envoyés par l'implant vers le programmateur, ils donnent naissance à une tension d'amplitude C aux bornes de la bobine réceptrice 68. C et D sont proportionnels, le facteur de proportionnalité dépendant de la distance entre la tête de programmation et l'implant :

$$C = P(d).D$$

Le bruit, qu'il est important de prendre en compte ici compte tenu des niveaux d'émission beaucoup plus faibles de l'implant, donne naissance à une tension d'amplitude $C_b$ aux bornes de la bobine 68. Le rapport signal/bruit intervient en effet de manière significative dans la transmission de l'implant vers le programmateur beaucoup plus que dans l'autre sens, comme le sait l'homme du métier, et c'est pourquoi ce paramètre doit être pris en compte pour déterminer si la transmission est possible ou non, et définir ainsi le niveau d'émission par l'implant.

Le rapport signal/bruit à la transmission d'un message 16 est :

$$RSB = C/C_b$$

À réception d'un message 16, le rapport signal/bruit calculé par 76 est analysé par le bloc de contrôle 72, qui vérifie que celui-ci est supérieur à un seuil minimal $\theta$.

La transmission dans le sens implant vers programmateur sera donc réputée possible si :

$$C/C_b > \theta.$$

On posera :

$$C_b - \theta = C_{min}.$$

Si une mesure donne un rapport signal/bruit supérieur à $\theta$ alors que l'amplitude aux bornes de la bobine 68 est supérieure à $C_{min}$, l'amplitude D correspondante du courant oscillent peut être multipliée par un facteur $\Psi^* = C_{min}/C$, pour obtenir un rapport signal/bruit juste égal à $\theta$.

$\Psi^*$ s'exprime en fonction du rapport signal/bruit mesuré :

$$\Psi^* = \theta/RSB.$$

Si $D_o$ est l'amplitude des courants générés par l'implant au début de la cession, une amplitude :

$$D_1 = \Psi^*.D_0.(1+u)$$

sera suffisante pour assurer une liaison correcte dans le sens implant vers programmateur (u étant un facteur de sécurité semblable à s).

Pour symétriser le système, on considère que la seule valeur $\Psi^*$ est transmise, dans un message 14, vers l'implant, mais c'est le programmateur qui, avantageusement, prendra en compte le calcul complet de $D_1$ et les amplitudes suivantes, ceci afin de ménager les ressources de calculs de l'implant.

Dans ce cas, l'amplitude $D_o$ ou, plus précisément, les amplitudes $D_n$ seront envoyées vers l'implant par le programmateur dans les messages 14.

Pour terminer, on remarquera que, comme on l'a illustré figure 2, les messages 16 ne véhiculent pas tous une valeur $\Phi^*$, de même que tous les messages 14 ne véhiculent pas de valeur $\Psi^*$. En effet, les instants séparant les calculs de ces valeurs dérivent des temps caractéristiques des mouvements humains dans une session d'interrogation d'un implant par télémétrie, temps qui peuvent être très longs devant la durée d'un message 14 ou 16. De la sorte, la transmission des informations $\Phi^*$ et $\Psi^*$ d'ajustement dynamique des niveaux d'émission de l'implant et/ou du programmateur sont ainsi sans incidence notable sur le débit des données de télémétrie échangées entre implant et programmateur.

## Revendications

1. Un ensemble comprenant, d'une part, un dispositif médical implantable actif, et, d'autre part, un appareil formant programmateur externe de ce dispositif implantable,

    le programmateur (12) comprenant des moyens (66, 70) d'émission à niveau variable de signaux (14) en direction du dispositif implantable et des moyens (68, 76) de réception de signaux (16) en provenance du dispositif implantable,
    le dispositif implantable (10) comprenant des moyens (26, 28) d'émission de signaux (16) en direction du programmateur et des moyens (26, 28) de réception de signaux (14) en provenance du programmateur, ainsi que des moyens de contrôle (32, 36) pour analyser le niveau du signal (A) reçu du programmateur et produire un signal de commande du niveau d'émission de ce dernier, ce signal (16) étant envoyé au programmateur par l'intermédiaire des moyens d'émission du dispositif implantable,

    **caractérisé en ce que** :

    - les moyens de contrôle du dispositif implantable comportent des moyens (36) de détection à sensibilité variable du signal reçu, comprenant des moyens atténuateurs programmables (60) commandables sélectivement, ainsi que des moyens à seuil (64),

- le programmateur opère initialement à un niveau d'émission maximal, puis
- les moyens de contrôle du dispositif implantable font varier de manière décroissante la sensibilité des moyens de détection jusqu'à franchissement du seuil des moyens à seuil,
- le dispositif implantable émet vers le programmateur le paramètre de sensibilité des moyens à sensibilité variable en tant que signal de commande du niveau d'émission, et
- le niveau d'émission du programmateur est ajusté conformément à ce signal de commande.

2. Un ensemble comprenant, d'une part, un dispositif médical implantable actif, et, d'autre part, un appareil formant programmateur externe de ce dispositif implantable,

le dispositif implantable (10) comprenant des moyens (26, 28) d'émission à niveau variable de signaux (16) en direction du programmateur et des moyens (26, 28) de réception de signaux (14) en provenance du programmateur,

le programmateur (12) comprenant des moyens (66, 70) d'émission de signaux (14) en direction du dispositif implantable et des moyens (68, 76) de réception de signaux (16) en provenance du dispositif implantable, ainsi que des moyens de contrôle (72) pour analyser le niveau du signal reçu du dispositif implantable et produire un signal de commande du niveau d'émission de ce dernier, ce signal (14) étant envoyé au dispositif implantable par l'intermédiaire des moyens d'émission du programmateur.

caractérisé en ce que les moyens de contrôle du programmateur comportent :

- des moyens de mesure donnant une valeur d'amplitude du bruit du signal reçu du dispositif implantable,
- des moyens d'analyse du rapport signal/ bruit du signal reçu du dispositif implantable, et
- des moyens d'asservissement du niveau d'émission du dispositif implantable, de manière à maintenir le rapport signal/bruit analysé au-dessus d'un seuil minimal donné.

**Patentansprüche**

1. Anordnung, umfassend einerseits eine aktive, implantierbare, medizinische Vorrichtung und anderseits einen Apparat, welcher ein externes Programmiergerät für diese implantierbare Vorrichtung bildet,
wobei das Programmiergerät (12) Mittel (66, 70) zum Aussenden eines variablen Niveaus von Signalen (14) in Richtung der implantierbaren Vorrichtung umfasst und Mittel (68, 76) zum Empfang von Signalen (16), die von der implantierbaren Vorrichtung herrühren,
wobei die implantierbare Vorrichtung (10) Mittel (26, 28) zum Aussenden von Signalen (16) in Richtung des Programmiergeräts und Mittel (26, 28) zum Empfang von Signalen (14) von dem Programmiergerät herrührend umfasst, wie auch Mittel zur Steuerung (32, 36) zum Analysieren des Niveaus des Signals (A), das von dem Programmiergerät empfangen wurde, und um ein Signal zur Steuerung des Sendeniveaus des letzteren zu produzieren, wobei dieses Signal (16) zu dem Programmiergerät geschickt wird über die Mittel zum Aussenden der implantierbaren Vorrichtung,
**dadurch gekennzeichnet, dass**:

- die Mittel zur Steuerung der implantierbaren Vorrichtung Mittel (36) zur Erfassung des empfangenen Signals mit variabler Empfindlichkeit aufweisen, die programmierbare Dämpfungsmittel (60) umfassen, die selektiv steuerbar sind, wie auch Schwellwertmittel (64),
- das Programmiergerät anfänglich auf einem maximalen Emissionsniveau arbeitet, dann
- die Mittel zur Steuerung der implantierbaren Vorrichtung in abnehmender Weise die Empfindlichkeit der Erfassungsmittel variieren lassen, bis zur Überschreitung des Schwellwerts der Schwellwertmittel,
- die implantierbare Vorrichtung zu dem Programmiergerät den Parameter der Empfindlichkeit der Mittel variabler Empfindlichkeit als Signal zur Steuerung des Sendeniveaus sendet, und
- das Sendeniveau des Programmiergeräts wird angepasst, übereinstimmend mit diesem Steuersignal.

2. Ensemble, umfassend einerseits eine aktive, implantierbare, medizinische Vorrichtung und anderseits einen Apparat, welcher ein externes Programmiergerät dieser implantierbaren Vorrichtung bildet,
wobei die implantierbare Vorrichtung (10) Mittel (26, 28) zum Senden von Signalen (16) bei variablem Niveau in Richtung des Programmiergeräts und Mittel (26, 28) zum Empfang von Signalen (14) umfasst, die von dem Programmiergerät herrühren,
wobei das Programmiergerät (12) Mittel (66, 70) zum Senden von Signalen (14) in Richtung der implantierbare Vorrichtung umfasst und Mittel (68, 76) zum Empfang von Signalen (16), die von der implantierbaren Vorrichtung herrühren, wie auch Mittel zur Steuerung (72) zum Analysieren des Niveaus des Signals, das von der implantierbaren Vorrichtung empfangen wurde, und Produzieren eines Signals zur Steuerung des Sendeniveaus der letzteren, wobei dieses Signal (14) zu der implantierbaren Vorrichtung geschickt wird über die Sendemittel des Programmiergeräts,

**dadurch gekennzeichnet, dass** die Mittel zur Steuerung des Programmiergeräts umfassen:

- Mittel zum Messen, welche einen Amplitudenwert des Rauschens des Signals ausgeben, das von der implantierbaren Vorrichtung empfangen wurde,
- Mittel zur Analyse des Signal-Rausch-Verhältnisses des Signals, das von der implantierbaren Vorrichtung empfangen wurde, und
- Mittel zur Einstellung des Sendeniveaus der implantierbaren Vorrichtung, um das Signal-Rausch-Verhältnis beizubehalten, das oberhalb einer gegebenen minimalen Schwelle analysiert wurde.

**Claims**

1. A set including, on the one hand, an active implantable medical device and, on the other hand, an apparatus consisting in an external programmer of said implantable device,
   the programmer (12) comprising means (66, 70) for transmitting signals (14) at a variable level towards the implantable device and means (68, 76) for receiving signals (16) originating from the implantable device,
   the implantable device (10) comprising means (26, 28) for transmitting signals (16) towards the programmer and means (26, 28) for receiving signals (14) originating from the programmer, as well as control means (32, 36) for analysing the level of the signal (A) received from the programmer and for issuing a signal for controlling the transmission level of the latter, said signal (16) being sent to the programmer via the transmitting means of the implantable device,
   **characterised in that**:

   - the control means of the implantable device includes variable sensitivity means (36) for detecting the received signal, including selectively operable programmable attenuator means (60), as well as threshold means (64),
   - the programmer initially operates at maximum transmission level, then
   - the control means of the implantable device decreasingly vary the sensitivity of the detection means until the threshold of the threshold means is crossed,
   - the implantable device transmits to the programmer the sensitivity parameter of the variable sensitivity means as a signal for controlling the transmission level, and
   - the transmission level of the programmer is adjusted according to said control signal.

2. A set including, on the one hand, an active implantable medical device and, on the other hand, an apparatus consisting in an external programmer of said implantable device,
   the implantable device (10) comprising means (26, 28) for transmitting signals (16) at a variable level towards the programmer and means (26, 28) for receiving signals (14) originating from the programmer,
   the programmer (12) comprising means (66, 70) for transmitting signals (14) towards the implantable device and means (68, 76) for receiving signals (14) originating from the programmer, as well as control means (72) for analysing the level of the signal (A) received from the implantable device and for issuing a signal for controlling the transmission level of the latter, said signal (14) being sent to the implantable device via the transmitting means of the programmer,
   **characterised in that** the control means of the programmer includes :

   - measurement means issuing a value of amplitude of the noise of the signal received from the implantable device,
   - means for analysing the signal-to-noise ratio of the signal received from the implantable device, and
   - servo means for the transmission level of the implantable device, in order to maintain the analysed signal-to-noise ration over a given minimum threshold.

BLOC
D'EMISSION

40

30

52

26

28

44

42

50

46

48

34

**FIG_3**

16

18

14

10

22

12

24

20

**FIG_1**

Vers bobine 26

54

62

(φ)

52

56

36

60

61

64

58

30

38

**FIG_4**

FIG_2

EP 0 850 663 B1